# EUROPEAN PATENT SPECIFICATION

(11) **EP 1 613 328 B1**
(45) Date of publication and mention of the grant of the patent: **18.03.2009**
(21) Application number: 04724599.8
(22) Date of filing: 31.03.2004
(51) Int. Cl.: A61K 31/55, A61P 25/14

(54) **USE OF CARBAMAZEPINE DERIVATIVES FOR THE TREATMENT OF AGITATION IN DEMENTIA PATIENTS**
VERWENDUNG VON CARBAMAZEPINDERIVATEN ZUR BEHANDLUNG VON AGITATIO IN DEMENSPATIENTEN
UTILISATION DE DERIVES DE CARBAMAZEPINE DANS LE TRAITEMENT DE L'AGITATION CHEZ DES PATIENTS ATTEINTS DE DEMENCE

(30) Priority: 01.04.2003 US 459338 P
(43) Date of publication of application: 11.01.2006
(73) Proprietor: NOVARTIS AG, 4056 Basel (CH); Novartis Pharma GmbH, 1230 Wien (AT)
(72) Inventor: SHUA-HAIM, Joshua, Eatontown, NJ 07724 (US); MARTENYI, Ferenc, H-1061 Budapest (HU)
(74) Representative: Harrison Goddard Foote
(86) International application number: PCT/EP2004/003418
(87) International publication number: WO 2004/087166

(56) References cited:
- EP-A- 0 751 129
- WO-A-02/098418
- AMBROSIO A F ET AL: "MECHANISMS OF ACTION OF CARBAMAZEPINE AND ITS DERIVATIVES, OXCARBAZEPINE, BIA 2-093, AND BIA 2-024" NEUROCHEMICAL RESEARCH, PLENUM PRESS, NEW YORK, US, vol. 27, no. 1/2, February 2002 (2002-02), pages 121-130, XP009022844 ISSN: 0364-3190
- BENES J ET AL: "Anticonvulsant and Sodium Channel-Blocking Properties of Novel 10,11-Dihydro-5H-dibenz[b,f]azepine-5-carb oxamide Derivatives" JOURNAL OF MEDICINAL CHEMISTRY, AMERICAN CHEMICAL SOCIETY. WASHINGTON, US, vol. 42, 1999, pages 2582-2587, XP002206156 ISSN: 0022-2623
- LEMKE MATTHIAS R: "Effect of carbamazepine on agitation in Alzheimer's inpatients refractory to neuroleptics" JOURNAL OF CLINICAL PSYCHIATRY, vol. 56, no. 8, 1995, pages 354-357, XP000903495 ISSN: 0160-6689
- FITZGERALD BRIAN J ET AL: "Elevation of carbamazepine-10,11-epoxide by quetiapine." PHARMACOTHERAPY, vol. 22, no. 11, November 2002 (2002-11), pages 1500-1503, XP009033502 ISSN: 0277-0008
- SHUA-HAIM J: "Oxcarbazepine in the management of behavioral agitation in community-dwelling patients with Alzheimer's disease: An open-label Study." EUROPEAN NEUROPSYCHOPHARMACOLOGY, vol. 13, no. Supplement 4, September 2003 (2003-09), page S434, XP002288071 & 16TH CONGRESS OF THE EUROPEAN COLLEGE OF NEUROPSYCHOPHARMACOLOGY; PRAGUE, CZECH REPUBLIC; SEPTEMBER 20-24, 2003 ISSN: 0924-977X
- S.C. SCHACHTER: "Oxcabazepine: current status and clinical applications" EXP. OPIN. INVEST. DRUGS, vol. 8, no. 7, 1999,

## Description

The present invention relates to new pharmaceutical uses of the carboxamides of formula I (see below). The term "carboxamides" as used herein includes, but is not limited to oxcarbazepine, 10-hydroxy-10,11-dihydrocarbamazepine and 10-acetoxy-10,11-dihydrocarbamazepine.

In particular, the invention relates to the carbamazepine derivatives of formula I 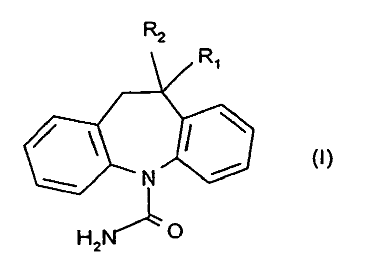 wherein (a) R₁ and R₂ together form an oxo group or (b) R₁ is hydrogen and R₂ is hydroxy or acetoxy, and to the pharmaceutically acceptable salts thereof.

If R₁ is hydrogen and R₂ is hydroxy or acetoxy, the compounds of formula I exist in two different enantiomers. The present invention relates to all such possible enantiomers.

The compound of formula I wherein R₁ and R₂ together form an oxy group is known as oxcarbazepine (10-oxo-10,11-dihydro-5H-dibenz(b,f)azepine-5-carboxamide). The preparation of oxcarbazepine and its pharmaceutically acceptable salts is described, e.g., in the German patent 2,011,087. Oxcarbazepine is marketed under the brand TRILEPTAL^{™}. It is a known anticonvulsant drug useful in the treatment of seizures of, for example, epileptic origin.

The preparation of the compound of formula I wherein R₁ is hydrogen and R₂ is hydroxy and its pharmaceutically acceptable salts is described, e.g., in US 3,637,661. The preparation of the compound of formula I wherein R₁ is hydrogen and R₂ is acetoxy and its pharmaceutically acceptable salts is described, e.g., in US 5,753,646. Both compounds are described to be efficacious against epilepsy.

In accordance with the present invention, it has now surprisingly been found that the compound of formula I and the pharmaceutically acceptable salts thereof are also useful for the treatment of agitation, in particular behavioral agitation, in dementia patients, especially in patients with Alzheimer's disease.

Agitation is commonly associated with dementia and contributes to diminished quality of life for patients as well as their caregivers.

The term "dementia" as used herein relates to a condition which can be characterized as a loss, usually progressive, of cognitive and intellectual functions, without impairment of perception or consciousness caused by a variety of disorders including severe infections and toxins, but most commonly associated with structural brain disease. Characterized by disorientation, impaired memory, judgment and intellect and a shallow labile affect. The term "dementia" includes, but is not restricted to AIDS dementia, Alzheimer dementia, presenile dementia, senile dementia, catatonic dementia, dialysis dementia (dialysis encephalopathy syndrome), epileptic dementia, hebephrenic dementia, Lewy body dementia (diffuse Lewy body disease), multi-infarct dementia (vascular dementia), paralytic dementia, posttraumatic dementia, dementia praecox, primary dementia, toxic dementia and vascular dementia.

In an 8-week, open-label, single-arm study are recruited twenty-six patients community-dwelling elderly patients (>63 years of age) diagnosed with Alzheimer's disease. After a 1-week screening period to assess each patient's agitation level, oxcarbazepine 150 mg/day treatment is initiated and titrated by 150 mg/day every 3-7 days to a maximum of 1200 mg/day with a mean oxcarbazepine daily dose of 340 mg/day (range 150-552 mg/day). The primary outcome measure is improvement in behavioral agitation, based on the 38-item Cohen-Mansfield Agitation Inventory (CMAI) that evaluates the prevalence of pathologic and disruptive agitated behavior. Secondary evaluations includes effects on neuropsychiatric symptoms, cognitive impairment, global functioning, and tolerability.

Significant reductions in CMAI score from baseline (mean±SD: 60.6±14.7) occur at each timepoint (mean±SD change from baseline on Day 14: -16.9±19.7. Day 28: -22.6±19.0, Day 56: -20.8±19.6; all p≤0.0002). Significant improvement in mean Neuropsychiatric Inventory score also occur at each timepoint (all p≤0.02 versus screening). Cognitive performance and global functioning remain stable throughout the study.

As shown by this this open-label, prospective study, compounds of formula I, especially oxcarbazepine, are effective and generally well tolerated in the treatment of agitation, in particular in patients with Alzheimer's disease.

For the treatment of the conditions mentioned herein, appropriate dosage will of course vary depending upon, for example, the host, the mode of administration, the specific compound or enantiomer used and the nature and severity of the condition being treated. In larger mammals, for example humans, an indicated daily dosage is in the range from about 50 to about 2000 mg, preferably from about 100 to about 1200 mg, more preferably from about 150 to about 600 mg, e.g. 340 mg, of a compound according to the invention conveniently administered, for example, in divided doses up to four times a day.

The compounds may be administered in any usual manner, e.g. orally, for example in the form of tablets or capsules, or parenterally, for example in the form of injection solutions or suspensions.

The present invention also provides pharmaceutical compositions comprising the compounds in association with at last one pharmaceutical carrier or diluent for use in the treatment of agitation. Such compositions may be manufactured in conventional manner.

Unit dosage forms may contain for example from about 2.5 mg to about 1000 mg of the compound, preferably about 300 or 600 mg.

Oxcarbazepine can be administered, e.g., in the form as it is marketed, e.g. under the trademark TRILEPTAL^{™}.

The invention further provides the use of a compound of formula I for the manufacture of a pharmaceutical composition for the treatment of agitation.

The invention further provides a compound of formula I for use in the treatment of agitation.

For the treatment of agitation a compound of formula I can be administered alone or in combination with a nootropic agent.

The term "nootropic agent" as used herein includes, but is not limited to nootropic plant extracts, calcium antagonists, cholinesterase inhibitors, dihydroergotoxin, nicergoline, piracetame, purine derivates, pyritinol, vincamine and vinpocetine.

The term "nootropic plant extracts" as used herein includes, but is not limited to extracts from Ginkgo leafs. The term "calcium antagonists" as used herein includes, but is not limited to cinnarizine and nimodipine. The term "cholinesterase inhibitors" as used herein includes, but is not limited to donepezil hydrochloride, rivastigmine and galantamine hydrobromide. The term "purine derivates" as used herein includes, but is not limited to pentifyllin.

Extracts from Ginkgo leafs can be administered, e.g., in the form as marketed, e.g. under the trademark Ginkodilat^{™} according to the information provided by the package insert. Cinnarizine can be administered, e.g., in the form as marketed, e.g. under the trademark Cinnarizin forte-ratiopharm^{™}. Nimodipine can be administered, e.g., in the form as marketed, e.g. under the trademark Nimotop^{™}. Donepezil hydrochloride can be administered, e.g., in the form as marketed, e.g. under the trademark Aricept^{™}. Rivastigmine can be prepared as disclosed in US 5,602,176. It can be administered, e.g., in the form as marketed, e.g. under the trademark Exelon^{™}. Galantamine hydrobromide can be administered, e.g., in the form as marketed, e.g. under the trademark Reminyl^{™}. Dihydroergotoxin can be administered, e.g., in the form as marketed, e.g. under the trademark Hydergin^{™}. Nicergoline can be administered, e.g., in the form as marketed, e.g. under the trademark Sermion^{™}. Piracetam can be administered, e.g., in the form as marketed, e.g. under the trademark Cerebroforte^{™}. Pentifyllin can be administered, e.g., in the form as marketed, e.g. under the trademark Cosaldon^{™}. Pyritinol can be administered, e.g., in the form as marketed, e.g. under the trademark Encephabol^{™}. Vinpocetin can be administered, e.g., in the form as marketed, e.g. under the trademark Cavinton^{™}.

The structure of the active agents identified by code nos., generic or trade names may be taken from the actual edition of the standard compendium "The Merck Index" or from databases, e.g. Patents international (e.g. IMS World Publications). The corresponding content thereof is hereby incorporated by reference.

Hence, the present invention pertains also to a combination comprising a compound of the invention, and at least one compound selected from the group consisting of nootropic plant extracts, cholinesterase inhibitors, dihydroergotoxin, nicergoline, piracetame, purine derivates, pyritinol, vincamine and vinpocetine, in which the active ingredients are present in each case in free form or in the form of a pharmaceutically acceptable salt and optionally at least one pharmaceutically acceptable carrier, for simultaneous, separate or sequential use, especially for use in a method of treating agitation.

Such a combination can be a combined preparation or a pharmaceutical composition.

The term "a combined preparation", as used herein defines especially a "kit of parts" in the sense that the active ingredients as defined above can be dosed independently or by use of different fixed combinations with distinguished amounts of the ingredients, i.e., simultaneously or at different time points. The parts of the kit can then, e.g., be administered simultaneously or chronologically staggered, that is at different time points and with equal or different time intervals for any part of the kit of parts. Preferably, the time intervals are chosen such that the effect on the treated disease in the combined use of the parts is larder than the effect which would be obtained by use of only any one of the active ingredients.

Hence, the present invention also provides
- the use of a combination as disclosed herein for the preparation of a medicament for the treatment of agitation, in particular behavioral agitation, in dementia patients, especially in patients with Alzheimer's disease; and
- a commercial package comprising a combination as disclosed herein together with instructions for simultaneous, separate or sequential use thereof in the treatment of agitation, in particular behavioral agitation, in dementia patients, especially in patients with Alzheimer's disease.

In one preferred embodiment of the invention, the combination partner (b) is a cholinesterase inhibitor, e.g. rivastigmine.

Unless mentioned otherwise herein, the following dosages of the combination partners (b) can be administered to the patient:

Cinnarizine may be administered to a patient in a total daily dosage of between about 75 to about 150 mg.

Nimodipine may be administered to a patient in a total daily dosage of between about 60 to about 120 mg.

Donepezil hydrochloride may be administered to a patient in a total daily dosage of between about 5 mg and 10 mg.

Rivastigmine may be administered to a patient in a total daily dosage of between about 6 and about 12 mg.

Galantamine may be administered to a patient in a total daily dosage of between about 12 and 24 mg, e.g. 12 mg twice daily.

Dihydroergotoxin may be administered in the form of its methansulfonate to a patient in a total daily dosage of between about 4 mg and 10 mg, e.g. about 8 mg.

Nicergoline may be administered in the form of its tartrate by intramuscular injection to a patient in a total daily dosage of between about 4 mg and 8 mg.

Piracetam may be administered to a patient in a total daily dosage of between about 1200 and 5000 mg, e.g. 4800 mg/day.

Pentifyllin may be administered to a patient in a total daily dosage of between about 400 and 800 mg.

Pyritinol may be administered in the form of its hydrochloride to a patient in a total daily dosage of about 600 mg.

Vinpocetin may be administered to a patient in a total daily dosage of between about 10 and 15 mg.

## Claims

1. The use of a compound of formula 1 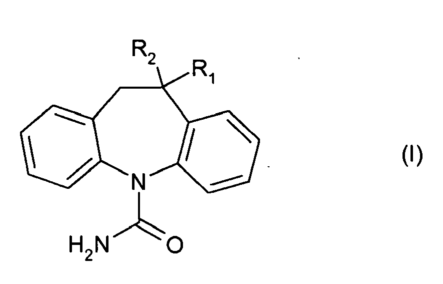 wherein (a) R₁ and R₂ together form an oxo group or (b) R₁ is hydrogen and R₂ is hydroxy or acetoxy, or a pharmaceutically acceptable salt thereof, for the manufacture of a pharmaceutical composition for the treatment of agitation.

2. The use according to claim 1 wherein the disease is behavioral agitation.

3. The use according to claim 1 or claim 2 wherein the pharmaceutical composition is for the treatment of agitation in dementia patients.

4. The use according to any of claims 1 to 3, wherein the patient to be treated is diagnosed to have Alzheimer's disease.

5. A compound for use in the treatment of agitation, namely a compound of formula 1 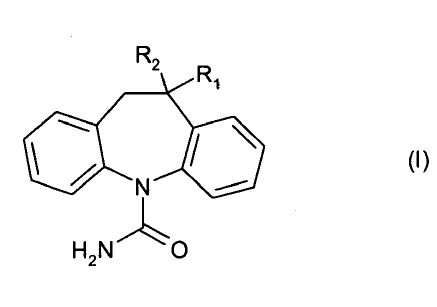 wherein (a) R₁ and R₂ together form an oxy group or (b) R₁ is hydrogen and R₂ is hydroxy or acetoxy,
or a pharmaceutically acceptable salt thereof.

6. A compound according to claim 5, wherein the compound of formula I is oxcarbazepine.

7. A compound according to claim 5 or claim 6, wherein the disease is behavioral agitation.

8. A compound according to claim 5 or claim 6 which is for the treatment of agitation in dementia patients.

9. A compound according to any of claims 5 to 8, wherein the subject to be treated is diagnosed to have Alzheimer's disease.

10. A compound according to any of claims 5 to 9 for administration in combination with a nootropic agent.

11. A pharmaceutical composition which incorporates as active agent a compound as defined in claim 1 or a pharmaceutically acceptable salt thereof, for use in the treatment of agitation.

12. A combination comprising (a) a compound of formula 1 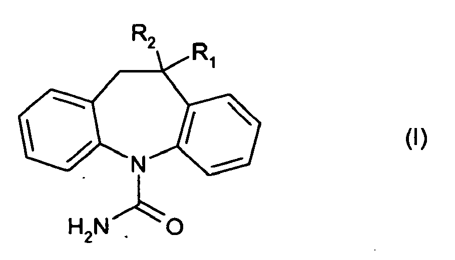 wherein (a) R₁ and R₂ together form an oxy group or (b) R¹ is hydrogen and R₂ is hydroxy or acetoxy,
and (b) at least one compound selected from nootropic plant extracts, cholinesterase inhibitors, dihydroergotoxin, nicergoline, piracetame, purine derivates, pyritinol, vincamine and vinpocetine, the active ingredients being present, in each case, in free form or in the form of a pharmaceutically acceptable salt and optionally at least one pharmaceutically acceptable carrier.

13. Combination according to claim 12, wherein the compound (b) is a cholinesterase inhibitor.

14. Use of a combination according to claim 12 or 13 for the preparation of a medicament for the treatment of agitation.

15. Use according to claim 14 wherein the agitation is in dementia patients.

16. A commercial package comprising a combination according to claim 12 or 13 together with instructions for simultaneous, separate or sequential use of the combination in the treatment of agitation.

17. A package according to claim 16 wherein the agitation is in dementia patients.

## Patentansprüche

1. Verwendung einer Verbindung der Formel 1 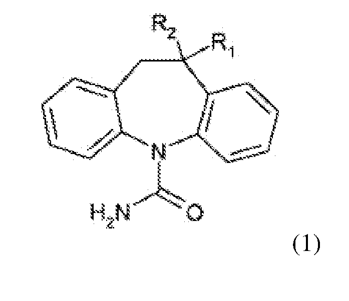 worin (a) R₁ und R₂ zusammen eine Oxo-Gruppe bilden; oder (b) R₁ für Wasserstoff steht und R₂ für Hydroxy oder Acetoxy steht, oder ein pharmazeutisch annehmbares Salz davon, für die Herstellung einer pharmazeutischen Zubereitung für die Behandlung von Unruhe.

2. Verwendung nach Anspruch 1, worin die Krankheit verhaltensbedingte Unruhe ist.

3. Verwendung nach Anspruch 1 oder Anspruch 2, worin die pharmazeutische Zubereitung für die Behandlung von Unruhe bei Demenz-Patienten dient.

4. Verwendung nach irgendeinem der Ansprüche 1 bis 3, worin dem zu behandelnden Patienten diagnostiziert wurde, dass er die Alzheimer'sche Krankheit hat.

5. Verbindung zur Verwendung bei der Behandlung von Unruhe nämlich eine Verbindung der Formel 1 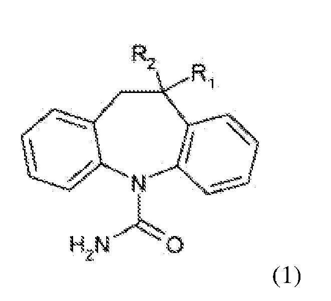 worin (a) R₁ und R₂ zusammen eine Oxo-Gruppe bilden; oder (b) R₁ für Wasserstoff steht und R₂ für Hydroxy oder Acetoxy steht, oder ein pharmazeutisch annehmbares Salz davon.

6. Verbindung nach Anspruch 5, worin die Verbindung der Formel 1 Oxcarbazepin ist.

7. Verbindung nach Anspruch 5 oder Anspruch 6, worin die Krankheit verhaltensbedingte Unruhe ist.

8. Verbindung nach Anspruch 5 oder Anspruch 6, welche der Behandlung von Unruhe bei Demenz-Patienten dient.

9. Verbindung nach irgendeinem der Ansprüche 5 bis 8, worin dem zu behandelnden Subjekt die Diagnose gestellt wurde, dass er die Alzerheimer'sche Krankheit hat.

10. Verbindung nach irgendeinem der Ansprüche 5 bis 9 zur Verabreichung in Kombination mit einem nootropen Mittel.

11. Pharmazeutische Zubereitung, welche als aktives Mittel eine Verbindung eingearbeitet aufweist, wie sie in Anspruch 1 definiert wurde oder ein pharmazeutisch annehmbares Salz davon, zur Verwendung bei der Behandlung von Unruhe.

12. Kombination, umfassend (a) eine Verbindung der Formel 1 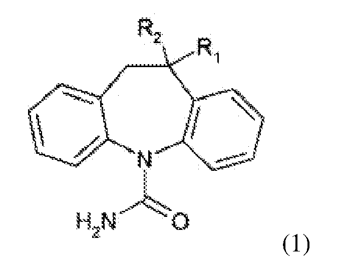 worin (a) R₁ und R₂ zusammen eine Oxo-Gruppe bilden; oder (b) R₁ für Wasserstoff steht und R₂ für Hydroxy oder Acetoxy steht;
und (b) wenigstens eine Verbindung, die gewählt ist aus nootropen PflanzenExtrakten, Cholinesterase-Inhibitoren, Dihydroergotoxin, Nicergolin, Piracetam, Purin-Derivaten, Pyritinol, Vincamin und Vinpocetin, wobei die aktiven Komponenten in jedem Fall in freier Form oder in Form eines pharmazeutisch annehmbaren Salzes zugegen sind, und gegebenenfalls wenigstens ein pharmazeutisch annehmbarer Träger.

13. Kombination nach Anspruch 12, worin die Verbindung (b) ein Cholinesterase-Inhibitor ist.

14. Verwendung einer Kombination nach Anspruch 12 oder 13 zur Herstellung eines Medikaments zur Behandlung von Unruhe.

15. Verwendung nach Anspruch 14, worin die Unruhe in Demenz-Patienten vorliegt.

16. Kommerzielle Packung, umfassend eine Kombination nach Anspruch 12 oder 13, zusammen mit Instruktionen für eine simultane, getrennte oder aufeinander folgende Verwendung der Kombination bei der Behandlung von Unruhe.

17. Paket nach Anspruch 16, worin die Unruhe in Demenz-Patienten vorliegt.

## Revendications

1. L'utilisation d'un composé de formule (I) 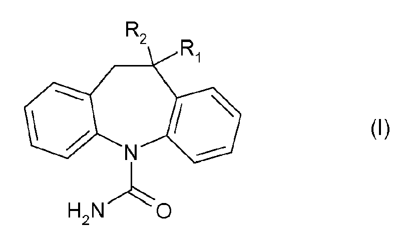 où (a) R₁ et R₂ constituent ensemble un groupe oxo ou (b) R₁ est un hydrogène et R₂ est un radical hydroxy ou acétoxy, ou d'un sel pharmaceutiquement acceptable de celui-ci, pour la fabrication d'une composition pharmaceutique pour le traitement de l'agitation.

2. L'utilisation selon la revendication 1, où la maladie est une agitation comportementale.

3. L'utilisation selon l'une des revendications 1 et 2, où la composition pharmaceutique est pour le traitement de l'agitation chez des patients atteints de démence.

4. L'utilisation selon l'une quelconque des revendications 1 à 3, où le patient à traiter est diagnostiqué avoir la maladie d'Alzheimer.

5. Un composé pour utilisation dans le traitement de l'agitation, à savoir un composé de formule (I) 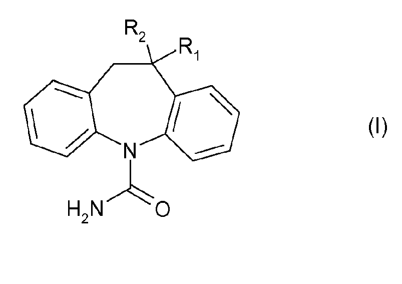 où (a) R₁ et R₂ forment ensemble un groupe oxo ou (b) R₁ est un hydrogène et R₂ est un radical hydroxy ou acétoxy, ou un sel pharmaceutiquement acceptable de celui-ci.

6. Un composé selon la revendication 5, où le composé de formule (I) est l'oxcarbazépine.

7. Un composé selon l'une des revendications 5 et 6, où la maladie est une agitation comportementale.

8. Un composé selon l'une des revendications 5 et 6 qui est pour le traitement de l'agitation chez des patients atteints de démence.

9. Un composé selon l'une quelconque des revendications 5 à 8, où le sujet devant être traité est diagnostiqué avoir la maladie d'Alzheimer.

10. Un composé selon l'une quelconque des revendications 5 à 9 pour administration en combinaison avec un agent nootropique.

11. Une composition pharmaceutique qui comprend en tant qu'agent actif un composé comme défini dans la revendication 1 ou un sel pharmaceutiquement acceptable de celui-ci, pour utilisation dans le traitement de l'agitation.

12. Une combinaison comprenant (a) un composé de formule (I) 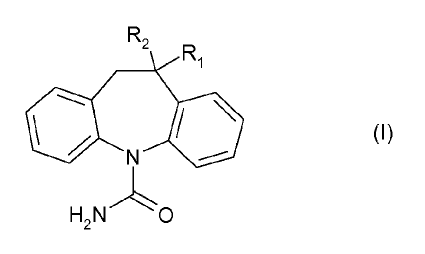 où (a) R₁ et R₂ forment ensemble un groupe oxo ou (b) R₁ est un hydrogène et R₂ est un radical hydroxy ou acétoxy,
et (b) au moins un composé choisi parmi des extraits de plantes nootropiques, des inhibiteurs de la cholinestérase, la dihydroergotoxine, la nicergoline, le piracétame, des dérivés de purine, le pyritinol, la vincamine et la vinpocétine, les substances actives étant présentes, dans chaque cas, sous forme libre ou sous la forme d'un sel pharmaceutiquement acceptable et optionnellement au moins un porteur pharmaceutiquement acceptable.

13. Combinaison selon la revendication 12, où le composé (b) est un inhibiteur de la cholinestérase.

14. Utilisation d'une combinaison selon l'une des revendications 12 et 13 pour la préparation d'un médicament pour le traitement de l'agitation.

15. Utilisation selon la revendication 14 où l'agitation est chez des patients atteints de démence.

16. Une présentation commerciale comprenant une combinaison selon l'une des revendications 12 et 13, avec des instructions pour l'utilisation simultanée, séparée ou séquentielle de la combinaison pour le traitement de l'agitation.

17. Une présentation selon la revendication 16, où l'agitation est chez des patients atteints de démence.
